(19) Europäisches Patentamt
European Patent Office
Office européen des brevets

(11) EP 0 713 704 A1

(12) **EUROPÄISCHE PATENTANMELDUNG**

(43) Veröffentlichungstag: **29.05.1996 Patentblatt 1996/22**

(51) Int. Cl.[6]: **A61K 31/54**, C07D 279/04

(21) Anmeldenummer: **95117500.9**

(22) Anmeldetag: **07.11.1995**

(84) Benannte Vertragsstaaten:
**AT BE CH DE ES FR GB IT LI NL SE**

(30) Priorität: **25.11.1994 DE 4442116**

(71) Anmelder: **HOECHST AKTIENGESELLSCHAFT**
**D-65926 Frankfurt am Main (DE)**

(72) Erfinder:
- **Strobel, Hartmut, Dr.**
**D-63755 Alzenau (DE)**
- **Bohn, Helmut, Dr.**
**D-61137 Schöneck (DE)**
- **Klemm, Peter, Dr.**
**D-65207 Wiesbaden (DE)**
- **Klingler, Otmar, Dr.**
**D-63110 Rodgau (DE)**
- **Schindler, Ursula, Dr.**
**D-65812 Bad Soden (DE)**
- **Schönafinger, Karl, Dr.**
**D-63755 Alzenau (DE)**
- **Zoller, Gerhard, Dr.**
**D-61137 Schöneck (DE)**

# (54) 2-Amino-1,3-thiazine als Hemmstoffe der Stickstoffmonoxid-Synthase

(57) Die vorliegende Erfindung betrifft 2-Amino-1,3-thiazine der allgemeinen Formel I,

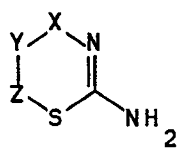

(I)

in der X für $CR^1R^2$, Y für $CR^{20}R^{21}$ und Z für $CR^{22}R^{23}$ steht und $R^1$, $R^2$, $R^{20}$, $R^{21}$, $R^{22}$ und $R^{23}$ die im Anspruch 1 angegebenen Bedeutungen haben, die Hemmstoffe der Stickstoffmonoxid-Synthase sind, und ihre Verwendung zur Behandlung von Krankheiten, die durch einen erhöhten Stickstoffmonoxid-Spiegel bedingt sind.

EP 0 713 704 A1

## Beschreibung

Die vorliegende Erfindung betrifft Thiazine der allgemeinen Formel I,

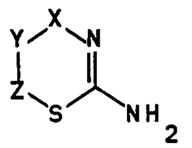

(I)

die aufgrund ihrer Fähigkeit zur Modulation der körpereigenen Stickstoffmonoxid-Produktion wertvolle Arzneimittel zur Vorbeugung und Bekämpfung von Krankheitszuständen sind, die durch einen gestörten Stickstoffmonoxid-Spiegel gekennzeichnet sind.

Stickstoffmonoxid (NO) spielt in verschiedensten physiologischen Prozessen eine bedeutende Rolle (siehe z.B. R.Henning, Nachr. Chem. Tech. Lab. 41 (1993), 413; J.F.Kerwin, M.Heller, Med. Res. Rev. 14 (1994), 23).

Es wirkt z.B. relaxierend auf die glatte Gefäßmuskulatur und ist auf diese Weise maßgeblich an der Regulation des Blutdrucks beteiligt, es steuert über eine Hemmung der Thrombocytenaggregation die Blutgerinnung, und es ist beispielsweise im Gehirn als Neurotransmitter in den Aufbau des Langzeitgedächtnisses involviert. In den NANC-Nerven des peripheren Nervensystems fungiert NO ebenfalls als Botenstoff. Die zelltoxische Wirkung des NO wird von Makrophagen für die Infektionsabwehr ausgenutzt.

Endogenes NO wird mit Hilfe mindestens drei verschiedener NO-Synthase-Isoenzyme aus Arginin gebildet.

Alle Isoenzyme benötigen NADPH, Flavinadenindinucleotid, Flavinmononucleotid und Tetrahydrobiopterin als Cofaktoren. Sie unterscheiden sich bezüglich ihrer Lokalisation im Organismus, ihrer Regulierbarkeit durch $Ca^{2+}$/Calmodulin sowie ihrer Induzierbarkeit durch Endotoxine und Cytokine. Die konstitutiven, calciumabhängigen NO-Synthasen finden sich beispielsweise im Endothel und im Gehirn und sind dort in die Regulation von Blutdruck und -gerinnung bzw. in Reizleitungsprozesse involviert. Die cytokin-induzierbare, calciumunabhängige Isoform tritt beispielsweise in Makrophagen, Glattmuskelzellen und Hepatocyten auf. Sie ist in der Lage, über einen langen Zeitraum relativ große Mengen NO zu produzieren und wird für entzündliche Prozesse und die zelltoxische Aktivität der Makrophagen verantwortlich gemacht.

Ein gestörter NO-Haushalt hat schwerwiegende Erkrankungen und Schädigungen zur Folge. So führt die exzessive Bildung von NO im septischen oder hämorrhagischen Schock zu massiven pathologischen Blutdruckabfällen. Übersteigerte NO-Produktion ist an der Entstehung von Typ-1-Diabetes und Atherosklerose beteiligt und scheint auch für die glutamatinduzierte Neurotoxizität nach cerebraler Ischämie verantwortlich zu sein. Hohe NO-Konzentrationen können darüberhinaus durch Desaminierung von Cytosin zu DNA-Schädigungen führen.

Der Ansatz, eine Modulation der NO-Produktion zur Behandlung dieser Krankheitsbilder zu verwenden, wurde bislang hauptsächlich mit Hilfe von Arginin-Analoga realisiert (GB-A-2 240 041; WO-A-93/13055; WO-A-93/24126; WO-A-94/02453; WO-A-94/07482). Als weitere potentielle NO-Synthase-Hemmstoffe werden in der Literatur N-Iminoethylornithin (McCall et al., Br.J.Pharmacol. 102 (1991), 234), Aminoguanidin (T.P.Misko et al., Eur.J.Pharmacol.

233 (1993), 119; EP-A-547588), Methylguanidin (US-A-5 246 971), Nitro- und Cyano-Aryle (WO-A-94/12163) sowie Pyrimidine, Pyridine, Pteridinone und Indazole (WO-A-94/14780) diskutiert. In der WO-A-94/12165 wird die hemmende Wirkung von Isothioharnstoffen auf die NO-Synthase offenbart. Neben den dort hauptsächlich untersuchten offenkettigen Isothioharnstoffen werden auch 2-Amino-1,3-thiazol-Derivate erwähnt, Verbindungen des Thiazintyps werden aber nicht explicit genannt.

Eine pharmakologische Wirkung verschiedener Thiazine ist bereits bekannt. So ist beispielsweise eine entzündungshemmende und immunotrope Wirksamkeit bestimmter Oxothiazine vorbeschrieben (Arch. Immunol. Ther. Exp. 26 (1978), 921; 26 (1978), 943; 29 (1981), 235). Antithyroide Wirkungen von 4-Oxo-thiazinen sind aus Farm. Zh. (Kiev) (1977), (5), 55-60, bekannt. N-Alkyl-Derivate von 2-Aminothiazinen sind als Inhibitoren der Indolamin-N-Methyl-Transferase in US-A-4 054 652 genannt. Die DE-A-2 241 140 beschreibt eine analgetische Wirkung bestimmter Spirothiazine. Eine blutdrucksteigernde Wirkung von 2-Amino-5,6-dihydro-4H-1,3-thiazin und dessen C-phenylierter Derivate ist aus Arch. Pharm. 301 (1968), 750 bekannt, sowie eine spezifisch ganglionär erregende Wirkung erstgenannter Verbindung aus Liebigs Ann. Chem. 614 (1958), 83. Nach B.G. Khadse et al., Bull. Haffkine Inst. 4 (1976), 113 besitzt 2-Amino-5,6-dihydro-4H-1,3-thiazin zudem eine schwache anthelminthische Wirkung.

Überraschend wurde nun gefunden, daß Thiazin-Derivate der allgemeinen Formel I besonders gut geeignete Hemmstoffe der induzierbaren NO-Synthase sind und die NO-Bildung wesentlich stärker hemmen als die verwandten Thiazol-Derivate. Sie sind somit als Arzneimittel bei Krankheiten geeignet, die durch einen überhöhten NO-Spiegel charakterisiert sind.

Gegenstand der vorliegenden Erfindung ist die Verwendung von 2-Amino-1,3-thiazinen der allgemeinen Formel I,

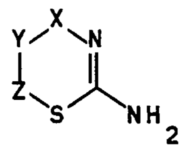

(I)

in der X für $CR^1R^2$ steht und
$R^1$ und $R^2$ unabhängig voneinander für Wasserstoff, Halogen, $R^3$, $R^4$, $OR^5$, $SR^{5'}$, $S(O)_p$-$R^6$, wobei p für 1 oder 2 steht, $NR^7R^8$, Cyan, Nitro, $COOR^9$, $CONR^{7'}R^{8'}$ oder $P(O)(OH)_2$ stehen; oder $R^1$ und $R^2$ zusammen für ($C_1$-$C_6$)-Alkyliden oder ($C_3$-$C_7$)-Cycloalkyliden stehen, die auch jeweils einen oder mehrere Reste $R^{10}$ tragen können, oder $R^1$ und $R^2$ zusammen mit dem Kohlenstoffatom, an das sie gebunden sind, einen Rest der allgemeinen Formel II

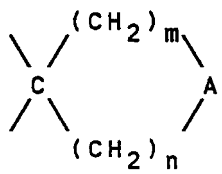

(II)

bilden, der vollständig oder teilweise dehydriert sein kann, der auch benzanelliert sein kann, der durch einen oder mehrere gleiche oder verschiedene Reste $R^{10}$ substituiert sein kann und worin m und n unabhängig voneinander für die Zahlen 0, 1, 2, 3, 4 oder 5 stehen, aber die Summe m + n einen der Werte 1, 2, 3, 4 oder 5 hat;
$R^3$ für ($C_1$-$C_{10}$)-Alkyl, ($C_2$-$C_{10}$)-Alkenyl, ($C_2$-$C_{10}$)-Alkinyl; ($C_3$-$C_7$)-Cycloalkyl, ($C_9$-$C_{11}$)-Benzocycloalkyl oder den Rest eines 4- bis 10-gliedrigen Heterocyclus mit einem bis vier gleichen oder verschiedenen Heteroatomen aus der Reihe Stickstoff, Sauerstoff und Schwefel steht, der aromatisch, teilweise gesättigt oder vollständig gesättigt sein kann, wobei der Alkyl-, Alkenyl-, Alkinyl-, Cycloalkyl- und der heterocyclische Rest durch einen oder mehrere gleiche oder verschiedene Reste $R^{10}$ substituiert sein können;
$R^4$ für Phenyl oder Naphthyl steht, die durch ein bis drei gleiche oder verschiedene Reste aus der Reihe ($C_1$-$C_4$)-Alkyl, ($C_1$-$C_4$)-Alkoxy, Halogen, Nitro, Trifluormethyl, Cyan, Hydroxy, ($C_1$-$C_4$)-Alkoxycarbonyl, Carboxyl, Carbamoyl, ($C_1$-$C_4$)-Alkyl-$S(O)_q$, wobei q für 0, 1 oder 2 steht, Amino, ($C_1$-$C_4$)-Alkylcarbonylamino, ($C_1$-$C_4$)-Alkylamino und Di(($C_1$-$C_4$)-Alkyl)amino substituiert sein können;
$R^5$ und $R^{5'}$ unabhängig voneinander für Wasserstoff, ($C_1$-$C_{10}$)-Alkyl, in dem die Alkylkette auch durch ein bis drei Sauerstoffatome unterbrochen sein kann, ($C_2$-$C_{10}$)-Alkenyl, ($C_3$-$C_7$)-Cycloalkyl, $R^4$, $R^4$-($C_1$-$C_6$)-Alkyl, den Rest eines 5- bis 7-gliedrigen Heterocyclus mit einem bis vier gleichen oder verschiedenen Heteroatomen aus der Reihe Stickstoff, Sauerstoff und Schwefel steht, der aromatisch, teilweise gesättigt oder vollständig gesättigt sein kann, ($C_1$-$C_{10}$)-Alkanoyl, $R^4$-CO, $R^4$-($C_1$-$C_6$)-Alkyl-CO, ($C_1$-$C_{10}$)-Alkyloxycarbonyl, $R^4$O-CO, $R^4$-($C_1$-$C_6$)-Alkoxy-CO oder einen Rest der allgemeinen Formel III

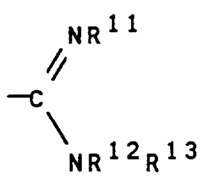

(III)

stehen;
$R^6$ für ($C_1$-$C_{10}$)-Alkyl, ($C_2$-$C_{10}$)-Alkenyl, $R^4$ oder $R^4$-($C_1$-$C_6$)-Alkyl steht;

$R^7$ und $R^8$ unabhängig voneinander die für $R^5$ angegebenen Bedeutungen haben oder für ($C_1$-$C_6$)-Alkylsulfonyl oder $R^4$-$S(O)_2$ stehen oder $R^7$ und $R^8$ zusammen mit dem Stickstoffatom, an das sie gebunden sind, einen Rest der allgemeinen Formel IV

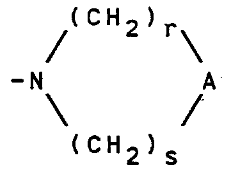

(IV)

bilden, der an den $CH_2$-Gruppen durch einen oder mehrere gleiche oder verschiedene Reste $R^{10}$ substituiert sein kann und worin r und s unabhängig voneinander für die Zahlen 1, 2, 3 oder 4 stehen, aber die Summe r + s kleiner als 6 ist;
$R^{7'}$ und $R^{8'}$ unabhängig von $R^7$ und $R^8$ die für $R^7$ und $R^8$ angegebenen Bedeutungen haben;
$R^9$ eine der Bedeutungen von $R^5$ hat, aber nicht für den Rest der allgemeinen Formel III steht;
$R^{10}$ für ($C_1$-$C_6$)-Alkyl, ($C_3$-$C_7$)-Cycloalkyl, den Rest eines 5- bis 7-gliedrigen Heterocyclus mit einem bis drei gleichen oder verschiedenen Heteroatomen aus der Reihe Stickstoff, Sauerstoff und Schwefel, der aromatisch, teilweise gesättigt oder vollständig gesättigt sein kann, $R^4$, Halogen, $OR^5$, $SR^{5'}$, $S(O)_p$-$R^6$, wobei p für 1 oder 2 steht, $NR^7R^8$, die Hydroximinogruppe, die Oxogruppe, Cyan, Nitro, $COOR^9$, $CONR^{7'}R^{8'}$ oder $P(O)(OH)_2$ steht;
die Reste $R^{11}$, $R^{12}$ und $R^{13}$ unabhängig voneinander für Wasserstoff, ($C_1$-$C_6$)-Alkyl, ($C_3$-$C_7$)-Cycloalkyl, $R^4$, ($C_1$-$C_6$)-Alkanoyl, ($C_1$-$C_6$)-Alkyl-$S(O)_2$ oder $R^4$-$S(O)_2$ stehen;
$R^{14}$ für ($C_1$-$C_4$)-Alkyl, Benzyl, Phenyl, ($C_1$-$C_6$)-Alkanoyl, ($C_1$-$C_6$)-Alkyl-$S(O)_2$ oder $R^4$-$S(O)_2$ steht;
A für eine Methylengruppe, die durch einen oder zwei gleiche oder verschiedene Reste $R^{10}$ substituiert sein kann, für Sauerstoff, Schwefel oder $NR^{14}$ steht;
Y für $CR^{20}R^{21}$ steht und $R^{20}$ und $R^{21}$ unabhängig von den Bedeutungen der Reste $R^1$ und $R^2$ die für $R^1$ und $R^2$ angegebenen Bedeutungen haben oder zusammen für =O, =N-OH oder =S stehen;
Z für $CR^{22}R^{23}$ steht und $R^{22}$ und $R^{23}$ unabhängig von den Bedeutungen der Reste $R^1$ und $R^2$ die für $R^1$ und $R^2$ angegebenen Bedeutungen haben;
wobei auch Reste der Gruppen X, Y und Z über eine Bindung verknüpft sein können, so daß ein Bicyclus oder Tricyclus gebildet wird;
und ihrer tautomeren Formen sowie ihrer pharmakologisch verträglichen Salze zur Vorbeugung und Behandlung von Krankheiten, die durch einen erhöhten Stickstoffmonoxid-Spiegel bedingt sind.

Halogen steht für Fluor, Chlor, Brom oder Iod, bevorzugt für Fluor oder Chlor.

Alkylgruppen können geradkettig oder verzweigt sein. Dies gilt auch, wenn sie in anderen Gruppen, beispielsweise in Alkoxy-, Alkylmercapto-, Alkyliden-, Alkoxycarbonyl- oder Alkanoylgruppen auftreten. Beispiele für Alkylgruppen, die in den erfindungsgemäß zu verwendenden Verbindungen der allgemeinen Formel I als solche, also als ($C_1$-$C_4$)-, ($C_1$-$C_6$)- oder ($C_1$-$C_{10}$)-Alkyl, oder in anderen Gruppen auftreten können, sind Methyl, Ethyl, n-Propyl, i-Propyl, n-Butyl, i-Butyl, sek-Butyl, tert-Butyl, n-Pentyl, 3-Methylbutyl, 2,2-Dimethylpropyl, n-Hexyl, n-Heptyl, n-Octyl, n-Nonyl oder n-Decyl. Bevorzugte Alkylgruppen sind Methyl, Ethyl, n-Propyl, i-Propyl, n-Butyl, i-Butyl und tert-Butyl.

Auch Alkenyl- und Alkinylreste können geradkettig oder verzweigt sein. Beispiele für Alkenylreste sind Vinyl, 1-Propenyl, Allyl, Butenyl, 3-Methyl-2-butenyl, für Alkinylreste Ethinyl, 1-Propinyl, Propargyl oder 5-Hexinyl.

Der ($C_3$-$C_7$)-Cycloalkylrest ist beispielsweise ein Cyclopropyl-, ein Cyclobutyl-, ein Cyclopentyl-, ein Cyclohexyl- oder ein Cycloheptylrest. Bevorzugte Cycloalkylreste sind der Cyclopentyl- und der Cyclohexylrest.

Der heterocyclische Rest mit 1, 2, 3 oder 4 Heteroatomen aus der Reihe Stickstoff, Sauerstoff und Schwefel kann aromatisch, teilweise gesättigt oder vollständig gesättigt sein und kann anelliert sein. Bevorzugte Ringgrößen des Heterocyclus sind 5-Ringe, 6-Ringe und 7-Ringe. Beispiele für Heterocyclen, von denen sich der Rest ableitet, sind Azetidin, Pyrrolidin, Pyrrol, Indol, Pyrazol, Imidazolidin, Imidazolin, Imidazol, 1,2,3-Triazol, 1,2,4-Triazol, Tetrazol, Tetrahydrofuran, Furan, 1,3-Dioxolan, Tetrahydrothiophen, Thiophen, Benzothiophen, 1,3-Dithiolan, 1,3-Oxazolin, 1,3-Oxazol, 1,3,4-Oxadiazol, Furazan, 1,3-Thiazolidin, 1,3-Thiazol, Piperidin, 1,2,5,6-Tetrahydropyridin, 1,4-Dihydropyridin, Pyridin, Chinolin, Isochinolin, Pyridazin, Pyrimidin, Piperazin, 1,2,3-Triazin, 1,3,5-Triazin, Perhydropyran, 1,3-Dioxan, 1,4-Dioxan, 1,3-Dithian, Dihydro-1,3-oxazin, Morpholin, Perhydro-1,4-thiazin, 5,6-Dihydro-4H-1,3-thiazin, Perhydroazepin, Pteridin und dessen Derivate. Die Reste können in beliebigen Positionen gebunden sein, Pyridylreste z.B. in der 2-, der 3- oder der 4-Position. Stickstoffheterocyclen können auch über ein Stickstoffatom gebunden sein.

Beispiele für Substituenten, die sich an Alkylresten und anderen Resten befinden können, sind Phenyl, Pyridyl, Methoxy, Hydroxy, Acetoxy, Acetyl, 2-Oxopropyl, Carboxy, Methoxycarbonyl, Ethoxycarbonyl, Methylmercapto, Methansulfonyl, Fluor, Aminocarbonyl, Diethylamino und Guanidino.

Beispiele für substituierte Alkylgruppen sind Benzyl, 2-Phenylethyl, Hydroxymethyl, Methoxycarbonylmethyl, Aminocarbonylmethyl und Diethylaminomethyl.

In monosubstituierten Phenylresten kann sich der Substituent in der 2-, der 3- oder der 4-Position befinden. Ist Phenyl zweifach substituiert, können die Substituenten in 2,3-, 2,4-, 2,5-, 2,6-, 3,4- oder 3,5-Position stehen.

Naphthylreste können als 1-Naphthyl- und 2-Naphthylreste vorliegen und ebenfalls in beliebigen Positionen substituiert sein. Beispiele für substituierte Derivate des als Arylrest bevorzugten Phenylrests sind 2-, 3- oder 4-Methylphenyl, 4-tert.-Butylphenyl, 2-, 3-oder 4-Methoxyphenyl, 3-Ethoxyphenyl, 2,3-, 3,4- oder 3,5-Dimethoxyphenyl, 3,4,5-Trimethoxyphenyl, 3-Aminophenyl, 3- oder 4-Dimethylaminophenyl, 4-Acetylaminophenyl, 2-, 3- oder 4-Fluorphenyl, 2,3- oder 3,4-Difluorphenyl, 2-, 3- oder 4-Chlorphenyl, 2,3-, 3,4-, 3,5- oder 2,6-Dichlorphenyl, 4-Bromphenyl, 4-Hydroxyphenyl, 3-Hydroxy-4-methoxyphenyl, 2-, 3- oder 4-Nitrophenyl, 3- oder 4-Trifluormethylphenyl, 2-, 3- oder 4-Cyanphenyl.

Für $R^5$ stehende Reste von Alkylketten, die durch Sauerstoffatome unterbrochen sind, sind beispielsweise 2-Methoxyethyl, 2-Ethoxyethyl, 2-(2-Methoxyethoxy)ethyl oder 2-(2-(2-Methoxyethoxy)ethoxy)ethyl.

Beispiele für Reste der allgemeinen Formel IV sind 1-Azetidinyl, Pyrrolidino, 2,5-Dimethylpyrrolidino, Piperidino, 2,6-Dimethylpiperidino, 2,2-Dimethylpiperidino, Morpholino, 1,4-Thiazin-4-yl, 3,3-Dimethyl-1,4-thiazin-4-yl, Piperazino, 4-Methylpiperazino, 4-Acetylpiperazino, 4-Methylsulfonylpiperazino, 4-Phenylpiperazino oder Azepino.

Die Verbindungen der allgemeinen Formel I können in verschiedenen tautomeren Formen und in verschiedenen stereoisomeren Formen vorliegen, so in der tautomeren Form der allgemeinen Formel V.

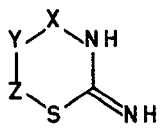

(V)

Die vorliegende Erfindung umfaßt nicht nur die Verwendung aller tautomeren Formen, sondern auch die aller stereoisomeren Formen, also z.B. die von reinen Enantiomeren, von Enantiomerengemischen und Racematen, von reinen Diastereomeren und Diastereomerengemischen oder von cis/trans- bzw. E/Z-Isomeren.

Geeignete Säuren für die Bildung pharmakologisch annehmbarer Säureadditionssalze der Verbindungen der allgemeinen Formel I sind beispielsweise: Chlorwasserstoff, Bromwasserstoff, Naphthalindisulfonsäuren, insbesondere Naphthalindisulfonsäure(1,5), Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Apfel-, Sulfamin-, Phenylpropion-, Glucon-, Ascorbin-, Isonicotin-, Methansulfon-, p-Toluolsulfon-, Zitronen- oder Adipinsäure. Die Säureadditionssalze können wie üblich durch Vereinigung der Komponenten, zweckmäßigerweise in einem geeigneten Lösungs- oder Verdünnungsmittel, hergestellt werden.

Verbindungen der allgemeinen Formel I, die eine saure Gruppe, beispielsweise eine Carbonsäuregruppe, enthalten, können an dieser mit anorganischen oder organischen Basen Salze bilden. Geeignete pharmakologisch annehmbare Salze sind beispielsweise Natriumsalze, Kaliumsalze, Magnesiumsalze, Calciumsalze, Ammoniumsalze oder Salze mit organischen Aminen, beispielsweise Ethanolamin oder Aminosäuren.

Bevorzugt ist die erfindungsgemäße Verwendung des Thiazins der allgemeinen Formel I, in der X, Y und Z gleichzeitig für die $CH_2$-Gruppe stehen.

Thiazine der allgemeinen Formel I sind zum Teil bekannt, pharmakologische Wirkungen oder medizinische Verwendungen sind aber bisher nur für wenige Verbindungen der allgemeinen Formel I bekannt. Für die bereits bekannten Verbindungen der allgemeinen Formel I, für die solche Wirkungen bisher nicht bekannt waren, stellt die vorliegende Erfindung die erste medizinische Indikation dar. Gegenstand der vorliegenden Erfindung sind auch 2-Amino-1,3-thiazine der allgemeinen Formel I

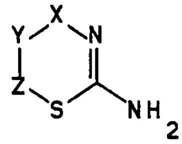

(I)

in der X für $CR^1R^2$ steht und

$R^1$ und $R^2$ unabhängig voneinander für Wasserstoff, Halogen, $R^3$, $R^4$, $OR^5$, $SR^{5'}$, $S(O)_p$-$R^6$, wobei p für 1 oder 2 steht, $NR^7R^8$, Cyan, Nitro, $COOR^9$, $CONR^{7'}R^{8'}$ oder $P(O)(OH)_2$ stehen; oder $R^1$ und $R^2$ zusammen für ($C_1$-$C_6$)-Alkyliden oder ($C_3$-$C_7$)-Cycloalkyliden stehen, die auch jeweils einen oder mehrere Reste $R^{10}$ tragen können, oder $R^1$ und $R^2$ zusammen mit dem Kohlenstoffatom, an das sie gebunden sind, einen Rest der allgemeinen Formel II

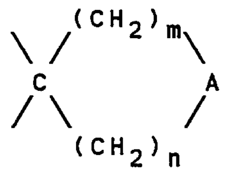

(II)

bilden, der vollständig oder teilweise dehydriert sein kann, der auch benzanelliert sein kann, der durch einen oder mehrere gleiche oder verschiedene Reste $R^{10}$ substituiert sein kann und worin m und n unabhängig voneinander für die Zahlen 0, 1, 2, 3, 4 oder 5 stehen, aber die Summe m + n einen der Werte 1, 2, 3, 4 oder 5 hat;

$R^3$ für ($C_1$-$C_{10}$)-Alkyl, ($C_2$-$C_{10}$)-Alkenyl, ($C_2$-$C_{10}$)-Alkinyl; ($C_3$-$C_7$)-Cycloalkyl, ($C_9$-$C_{11}$)-Benzocycloalkyl oder den Rest eines 4- bis 10-gliedrigen Heterocyclus mit einem bis vier gleichen oder verschiedenen Heteroatomen aus der Reihe Stickstoff, Sauerstoff und Schwefel steht, der aromatisch, teilweise gesättigt oder vollständig gesättigt sein kann, wobei der Alkyl-, Alkenyl-, Alkinyl-, Cycloalkyl- und der heterocyclische Rest durch einen oder mehrere gleiche oder verschiedene Reste $R^{10}$ substituiert sein können;

$R^4$ für Phenyl oder Naphthyl steht, die durch ein bis drei gleiche oder verschiedene Reste aus der Reihe ($C_1$-$C_4$)-Alkyl, ($C_1$-$C_4$)-Alkoxy, Halogen, Nitro, Trifluormethyl, Cyan, Hydroxy, ($C_1$-$C_4$)-Alkoxycarbonyl, Carboxyl, Carbamoyl, ($C_1$-$C_4$)-Alkyl-$S(O)_q$, wobei q für 0, 1 oder 2 steht, Amino, ($C_1$-$C_4$)-Alkylcarbonylamino, ($C_1$-$C_4$)-Alkylamino und Di(($C_1$-$C_4$)-Alkyl)amino substituiert sein können;

$R^5$ und $R^{5'}$ unabhängig voneinander für Wasserstoff, ($C_1$-$C_{10}$)-Alkyl, in dem die Alkylkette auch durch ein bis drei Sauerstoffatome unterbrochen sein kann, ($C_2$-$C_{10}$)-Alkenyl, ($C_3$-$C_7$)-Cycloalkyl, $R^4$, $R^4$-($C_1$-$C_6$)-Alkyl, den Rest eines 5- bis 7-gliedrigen Heterocyclus mit einem bis vier gleichen oder verschiedenen Heteroatomen aus der Reihe Stickstoff, Sauerstoff und Schwefel steht, der aromatisch, teilweise gesättigt oder vollständig gesättigt sein kann, ($C_1$-$C_{10}$)-Alkanoyl, $R^4$-CO, $R^4$-($C_1$-$C_6$)-Alkyl-CO, ($C_1$-$C_{10}$)-Alkyloxycarbonyl, $R^4$O-CO, $R^4$-($C_1$-$C_6$)-Alkoxy-CO oder einen Rest der allgemeinen Formel III

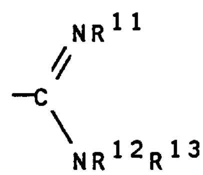

(III)

stehen;

$R^6$ für ($C_1$-$C_{10}$)-Alkyl, ($C_2$-$C_{10}$)-Alkenyl, $R^4$ oder $R^4$-($C_1$-$C_6$)-Alkyl steht;

$R^7$ und $R^8$ unabhängig voneinander die für $R^5$ angegebenen Bedeutungen haben oder für ($C_1$-$C_6$)-Alkylsulfonyl oder $R^4$-$S(O)_2$ stehen oder $R^7$ und $R^8$ zusammen mit dem Stickstoffatom, an das sie gebunden sind, einen Rest der allgemeinen Formel IV

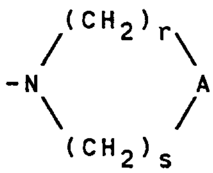

(IV)

bilden, der an den $CH_2$-Gruppen durch einen oder mehrere gleiche oder verschiedene Reste $R^{10}$ substituiert sein kann und worin r und s unabhängig voneinander für die Zahlen 1, 2, 3 oder 4 stehen, aber die Summe r + s kleiner als 6 ist;
$R^{7'}$ und $R^{8'}$ unabhängig von $R^7$ und $R^8$ die für $R^7$ und $R^8$ angegebenen Bedeutungen haben;
$R^9$ eine der Bedeutungen von $R^5$ hat, aber nicht für den Rest der allgemeinen Formel III steht;
$R^{10}$ für $(C_1$-$C_6)$-Alkyl, $(C_3$-$C_7)$-Cycloalkyl, den Rest eines 5- bis 7-gliedrigen Heterocyclus mit einem bis drei gleichen oder verschiedenen Heteroatomen aus der Reihe Stickstoff, Sauerstoff und Schwefel, der aromatisch, teilweise gesättigt oder vollständig gesättigt sein kann, $R^4$, Halogen, $OR^5$, $SR^{5'}$, $S(O)_p$-$R^6$, wobei p für 1 oder 2 steht, $NR^7R^8$, die Hydroximinogruppe, die Oxogruppe, Cyan, Nitro, $COOR^9$, $CONR^{7'}R^{8'}$ oder $P(O)(OH)_2$ steht;
die Reste $R^{11}$, $R^{12}$ und $R^{13}$ unabhängig voneinander für Wasserstoff, $(C_1$-$C_6)$-Alkyl, $(C_3$-$C_7)$-Cycloalkyl, $R^4$, $(C_1$-$C_6)$-Alkanoyl, $(C_1$-$C_6)$-Alkyl-$S(O)_2$ oder $R^4$-$S(O)_2$ stehen;
$R^{14}$ für $(C_1$-$C_4)$-Alkyl, Benzyl, Phenyl, $(C_1$-$C_6)$-Alkanoyl, $(C_1$-$C_6)$-Alkyl-$S(O)_2$ oder $R^4$-$S(O)_2$ steht;
A für eine Methylengruppe, die durch einen oder zwei gleiche oder verschiedene Reste $R^{10}$ substituiert sein kann, für Sauerstoff, Schwefel oder $NR^{14}$ steht;
Y für $CR^{20}R^{21}$ steht und $R^{20}$ und $R^{21}$ unabhängig von den Bedeutungen der Reste $R^1$ und $R^2$ die für $R^1$ und $R^2$ angegebenen Bedeutungen haben oder zusammen für =O, =N-OH oder =S stehen;
Z für $CR^{22}R^{23}$ steht und $R^{22}$ und $R^{23}$ unabhängig von den Bedeutungen der Reste $R^1$ und $R^2$ die für $R^1$ und $R^2$ angegebenen Bedeutungen haben;
wobei auch Reste der Gruppen X, Y und Z über eine Bindung verknüpft sein können, so daß ein Bicyclus oder Tricyclus gebildet wird;
und ihrer tautomeren Formen sowie ihrer pharmakologisch verträglichen Salze als pharmakologische Wirkstoffe, wobei aber $R^1$, $R^2$, $R^{20}$, $R^{21}$, $R^{22}$ und $R^{23}$ nicht gleichzeitig für Wasserstoff stehen können und, wenn $R^1$, $R^2$, $R^{20}$, $R^{21}$ und $R^{23}$ für Wasserstoff stehen, $R^{22}$ nicht für Phenyl stehen kann, und für den Fall, daß $R^{20}$, $R^{21}$, $R^{22}$ und $R^{23}$ für Wasserstoff stehen und $R^1$ und $R^2$ zusammen mit dem Kohlenstoffatom, an das sie gebunden sind, einen Rest der allgemeinen Formel II bilden, A nicht für Sauerstoff stehen kann, wenn m oder n 0 bedeutet und die Summe m + n 3 oder 4 ist.

Bestimmte der erfindungsgemäß als Hemmstoffe der NO-Synthase einsetzbare Thiazine der allgemeinen Formel I sind als solche in verschiedenen Veröffentlichungen beschrieben, z.B. N. Cohen et al., J. Org. Chem. 39 (1974), 1824; N. Cohen et al., J. Med. Chem. 21 (1978), 895; H. W. Schubert und O. Behner, Arch. Pharm. 301 (1968), 750 oder A. Schöberl et al., Liebigs Ann. Chem. 614 (1958), 83.

Gegenstand der vorliegenden Erfindung sind auch die bisher nicht beschriebenen Verbindungen der allgemeinen Formel I als solche, also 2-Amino-1,3-thiazine der allgemeinen Formel I,

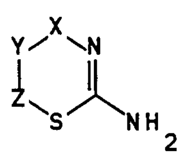

(I)

in der X für $CR^1R^2$ steht und
$R^1$ und $R^2$ unabhängig voneinander für Wasserstoff, Halogen, $R^3$, $R^4$, $OR^5$, $SR^{5'}$, $S(O)_p$-$R^6$, wobei p für 1 oder 2 steht, $NR^7R^8$, Cyan, Nitro, $COOR^9$, $CONR^{7'}R^{8'}$ oder $P(O)(OH)_2$ stehen; oder $R^1$ und $R^2$ zusammen für $(C_1$-$C_6)$-Alkyliden oder $(C_3$-$C_7)$-Cycloalkyliden stehen, die auch jeweils einen oder mehrere Reste $R^{10}$ tragen können, oder $R^1$ und $R^2$ zusammen mit dem Kohlenstoffatom, an das sie gebunden sind, einen Rest der allgemeinen Formel II

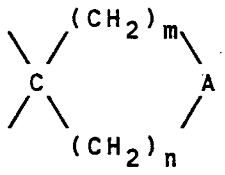

(II)

bilden, der vollständig oder teilweise dehydriert sein kann, der auch benzanelliert sein kann, der an den $CH_2$-Gruppen durch einen oder mehrere gleiche oder verschiedene Reste $R^{10}$ substituiert sein kann und worin m und n unabhängig voneinander für die Zahlen 0, 1, 2, 3, 4 oder 5 stehen, aber die Summe m + n einen der Werte 1, 2, 3, 4 oder 5 hat;

$R^3$ für $(C_1\text{-}C_{10})$-Alkyl, $(C_2\text{-}C_{10})$-Alkenyl, $(C_2\text{-}C_{10})$-Alkinyl; $(C_3\text{-}C_7)$-Cycloalkyl, $(C_9\text{-}C_{11})$-Benzocycloalkyl oder den Rest eines 4-bis 10-gliedrigen Heterocyclus mit einem bis vier gleichen oder verschiedenen Heteroatomen aus der Reihe Stickstoff, Sauerstoff und Schwefel steht, der aromatisch, teilweise gesättigt oder vollständig gesättigt sein kann, wobei der Alkyl-, Alkenyl-, Alkinyl-, Cycloalkyl- und der heterocyclische Rest durch einen oder mehrere gleiche oder verschiedene Reste $R^{10}$ substituiert sein können;

$R^4$ für Phenyl oder Naphthyl steht, die durch ein bis drei gleiche oder verschiedene Reste aus der Reihe $(C_1\text{-}C_4)$-Alkyl, $(C_1\text{-}C_4)$-Alkoxy, Halogen, Nitro, Trifluormethyl, Cyan, Hydroxy, $(C_1\text{-}C_4)$-Alkoxycarbonyl, Carboxyl, Carbamoyl, $(C_1\text{-}C_4)$-Alkyl-$S(O)_q$, wobei q für 0, 1 oder 2 steht, Amino, $(C_1\text{-}C_4)$-Alkylcarbonylamino, $(C_1\text{-}C_4)$-Alkylamino und Di($(C_1\text{-}C_4)$-Alkyl)amino substituiert sein können;

$R^5$ und $R^{5'}$ unabhängig voneinander für Wasserstoff, $(C_1\text{-}C_{10})$-Alkyl, in dem die Alkylkette auch durch ein bis drei Sauerstoffatome unterbrochen sein kann, $(C_2\text{-}C_{10})$-Alkenyl, $(C_3\text{-}C_7)$-Cycloalkyl, $R^4$, $R^4$-$(C_1\text{-}C_6)$-Alkyl, den Rest eines 5- bis 7-gliedrigen Heterocyclus mit einem bis vier gleichen oder verschiedenen Heteroatomen aus der Reihe Stickstoff, Sauerstoff und Schwefel steht, der aromatisch, teilweise gesättigt oder vollständig gesättigt sein kann, $(C_1\text{-}C_{10})$-Alkanoyl, $R^4$-CO, $R^4$-$(C_1\text{-}C_6)$-Alkyl-CO, $(C_1\text{-}C_{10})$-Alkyloxycarbonyl, $R^4$O-CO, $R^4$-$(C_1\text{-}C_6)$-Alkoxy-CO oder einen Rest der allgemeinen Formel III

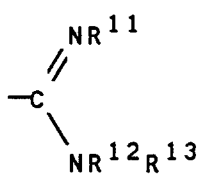

(III)

stehen;

$R^6$ für $(C_1\text{-}C_{10})$-Alkyl, $(C_2\text{-}C_{10})$-Alkenyl, $R^4$ oder $R^4$-$(C_1\text{-}C_6)$-Alkyl steht;

$R^7$ und $R^8$ unabhängig voneinander die für $R^5$ angegebenen Bedeutungen haben oder für $(C_1\text{-}C_6)$-Alkylsulfonyl oder $R^4$-$S(O)_2$ stehen oder $R^7$ und $R^8$ zusammen mit dem Stickstoffatom, an das sie gebunden sind, einen Rest der allgemeinen Formel IV

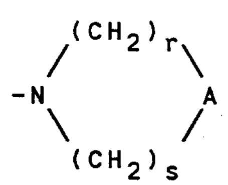

(IV)

bilden, der an den $CH_2$-Gruppen durch einen oder mehrere gleiche oder verschiedene Reste $R^{10}$ substituiert sein kann und worin r und s unabhängig voneinander für die Zahlen 1, 2, 3 oder 4 stehen, aber die Summe r + s kleiner als 6 ist;

$R^{7'}$ und $R^{8'}$ unabhängig von $R^7$ und $R^8$ die für $R^7$ und $R^8$ angegebenen Bedeutungen haben;

$R^9$ eine der Bedeutungen von $R^5$ hat, aber nicht für den Rest der allgemeinen Formel III steht;
$R^{10}$ für ($C_1$-$C_6$)-Alkyl, ($C_3$-$C_7$)-Cycloalkyl, den Rest eines 5- bis 7-gliedrigen Heterocyclus mit einem bis drei gleichen oder verschiedenen Heteroatomen aus der Reihe Stickstoff, Sauerstoff und Schwefel, der aromatisch, teilweise gesättigt oder vollständig gesättigt sein kann, $R^4$, Halogen, $OR^5$, $SR^{5'}$, $S(O)_p$-$R^6$, wobei p für 1 oder 2 steht, $NR^7R^8$, die Hydroximinogruppe, die Oxogruppe, Cyan, Nitro, $COOR^9$, $CONR^{7'}R^{8'}$ oder $P(O)(OH)_2$ steht;
die Reste $R^{11}$, $R^{12}$ und $R^{13}$ unabhängig voneinander für Wasserstoff, ($C_1$-$C_6$)-Alkyl, ($C_3$-$C_7$)-Cycloalkyl, $R^4$, ($C_1$-$C_6$)-Alkanoyl, ($C_1$-$C_6$)-Alkyl-$S(O)_2$ oder $R^4$-$S(O)_2$ stehen;
$R^{14}$ für ($C_1$-$C_4$)-Alkyl, Benzyl, Phenyl, ($C_1$-$C_6$)-Alkanoyl, ($C_1$-$C_6$)-Alkyl-$S(O)_2$ oder $R^4$-$S(O)_2$ steht;
A für eine Methylengruppe, die durch einen oder zwei gleiche oder verschiedene Reste $R^{10}$ substituiert sein kann, für Sauerstoff, Schwefel oder $NR^{14}$ steht;
Y für $CR^{20}R^{21}$ steht und $R^{20}$ und $R^{21}$ unabhängig von den Bedeutungen der Reste $R^1$ und $R^2$ die für $R^1$ und $R^2$ angegebenen Bedeutungen haben oder zusammen für =O, =N-OH oder =S stehen;
Z für $CR^{22}R^{23}$ steht und $R^{22}$ und $R^{23}$ unabhängig von den Bedeutungen der Reste $R^1$ und $R^2$ die für $R^1$ und $R^2$ angegebenen Bedeutungen haben;
wobei auch Reste der Gruppen X, Y und Z über eine Bindung verknüpft sein können, so daß ein Bicyclus oder Tricyclus gebildet wird;
und ihrer tautomeren Formen sowie ihrer pharmakologisch verträglichen Salze,
wobei aber,

a) wenn $R^{20}$, $R^{21}$, $R^{22}$ und $R^{23}$ für Wasserstoff stehen, nicht

a1) gleichzeitig $R^1$ für Wasserstoff und $R^2$ für Wasserstoff, Methyl, Ethoxy, Carboxy, Phenyl, Chlorphenyl, Dichlorphenyl, Methylphenyl, Dimethylphenyl, Nitrophenyl, Methoxyphenyl oder Acetylaminophenyl stehen kann;
a2) gleichzeitig $R^1$ für Methyl und $R^2$ für Phenyl oder 4-Methoxyphenyl oder gleichzeitig $R^1$ für Ethyl und $R^2$ für 3,4-Dimethoxyphenyl stehen kann;
a3) gleichzeitig $R^1$ und $R^2$ zusammen mit dem Kohlenstoffatom, an das sie gebunden sind, einen unsubstituierten Rest der allgemeinen Formel II bilden können, worin A für eine Methylengruppe und m und n beide für 2 stehen, oder einen Rest der allgemeinen Formel II bilden können, worin A für Sauerstoff und n für 0 steht;
a4) gleichzeitig $R^1$ und $R^2$ zusammen mit dem Kohlenstoffatom, an das sie gebunden sind, einen 6-Methoxy-1,2,3,4-tetrahydronaphthalin-1-yliden-Rest bilden können;

b) wenn $R^1$, $R^2$, $R^{20}$ und $R^{21}$ für Wasserstoff stehen, nicht

b1) gleichzeitig $R^{22}$ für Wasserstoff und $R^{23}$ für Methyl, Phenyl, Methoxyphenyl oder Brommethyl stehen kann;
b2) gleichzeitig $R^{22}$ und $R^{23}$ zusammen für $=CH_2$ stehen können;

c) wenn $R^1$, $R^2$, $R^{22}$ und $R^{23}$ für Wasserstoff stehen, nicht

c1) gleichzeitig $R^{20}$ für Wasserstoff und $R^{21}$ für Chlor, Hydroxy, Methylsulfonyl, n-Butylsulfonyl, Benzoyloxy, Phenyl oder Chlorphenyl stehen kann;
c2) gleichzeitig $R^{20}$ und $R^{21}$ zusammen für =O stehen können;

und wobei nicht gleichzeitig $R^1$, $R^{21}$, $R^{22}$ und $R^{23}$ für Wasserstoff stehen und $R^2$ und $R^{20}$ zusammen mit den beiden Kohlenstoffatomen, an die sie gebunden sind, einen 1,2-Cyclohexylen-Rest bilden können.

Die Synthese der erfindungsgemäß einsetzbaren Verbindungen der allgemeinen Formel I kann nach oder analog zu bekannten Verfahren erfolgen, z.B. durch Ringschluß von Halogenalkylisothiocyanaten nach H.W. Schubert und O. Behner, Arch. Pharm. 301 (1968) 750; durch Cyclisierung von Aminopropylthiocyanaten nach A. Schöberl et al., Liebigs Ann. Chem. 614 (1958), 83; durch Umsetzung von 3-Aminopropanolen bzw. 3-Aminopropylhalogeniden mit tert-Butylisothiocyanat nach A. Schöberl und K.-H. Magosch, Liebigs Ann. Chem. 742 (1970), 75, oder durch Cyclisierung von 3-Aminopropylisothioharnstoffen nach Fedoseev, Zh. Org. Khim. 8 (1972), 205. Die Angaben zur Synthese der Verbindungen der allgemeinen Formel I in den genannten Veröffentlichungen sind in vollem Umfang Bestandteil der vorliegenden Offenbarung.

Die erfindungsgemäße Hemmung der NO-Freisetzung durch die Verbindungen der allgemeinen Formel I kann durch einen Aktivitätsassay bestimmt werden, der auf Arbeiten von Bredt und Snyder sowie Schmidt et al. basiert (s. D.S. Bredt und S.S. Snyder, Isolation of nitric oxide synthase, a calmodulin-requiring enzyme, Proc. Natl. Acad. Sci. USA 87 (1990), 682; H.H.H.W.Schmidt et al., Purification of a soluble isoform of guanylyl cyclase-activating factor synthase, Proc. Natl. Acad. Sci. USA 88 (1991), 365). Hierbei wird für gereinigte NO-Synthase (NOS) das bei der NO-Bildung

anfallende Coprodukt L-Citrullin quantitativ erfaßt. Dies geschieht durch den Einsatz von $^3$H-radiomarkiertem L-Arginin als Substrat der Enzymreaktion, das zu $^3$H-L-Citrullin und NO umgesetzt wird. Nach Beendigung der Enzyminkubation wird entstandenes L-Citrullin von unverbrauchtem L-Arginin mittels Ionenaustauschchromatographie aus dem Reaktionsgemisch entfernt; die durch Flüssigkeitsszintillationsmessung ermittelte $^3$H-Aktivität entspricht dann der Menge an L-Citrullin.

Die Hemmung der NO-Freisetzung durch die Verbindungen der allgemeinen Formel I kann auch durch NO-Messung mittels Oxyhämoglobin auf Makrophagen wie folgt bestimmt werden:

Mäusemakrophagen (MM) des Stammes RAW 264.7 werden in Petrischalen (10 cm Durchmesser) angezüchtet. Als Nährmedium wird DMEM (Fa. Sigma Nr. D 5405), konditioniert mit 4 mM l-Glutamin, 3,5 g D-Glukose/l, 50 U/50 µg/ml Pen/Strep und 10 % FKS, eingesetzt unter 5 % $CO_2$, 95 % Luftfeuchtigkeit, 37°C. Die subkonfluenten Zellen werden mit dem Zellschaber abgekratzt, in Medium aufgenommen, die Zellzahl bestimmt (Zählung in Trypanblaulösung 0,2 %) und in 24-Napfplatten (1 x $10^6$ Zellen/ml, 1 ml/Napf) ausgesät.

Ca. 18 Stunden nach Aussaat werden die gut adherierten Mausmakrophagen mit LPS (Lipopolysaccharid von E. coli Serotyp 0111:B4, Fa. Sigma Nr. L 2630) und γ-Interferon (γIFN, Maus rekombinant, Firma Boehringer Mannheim Nr. 1276905) stimuliert. Dazu wird das Kulturmedium von den Zellen abgesaugt und 1 ml/Napf (24-Napfplatte) Inkubationsmedium (MEM ohne Phenolrot (Biochrom F0385)), konditioniert mit 4mM Glutamin, 3,5 g Glukose/l, 3,7 g $NaHCO_3$/l, 110 mg Natriumpyruvat/l, 50 U/50 µg/ml Pen/Strep und 5 % FKS, aufgegeben. Pro ml Inkubationsmedium werden 1 µl LPS und 1 µl γIFN pipettiert.

Daraus ergeben sich folgende Konzentrationen der Stimulatoren:

140 ng LPS/ml + 5 U γIFN/ml

Während der Stimulation über 4 Stunden wird die Neusynthese des NO-produzierenden Enzyms NO-Synthase (iNOS) induziert. Nach diesem Inkubationszeitraum wird das Inkubationsmedium abgesaugt und jeder Napf zweimal mit frischem Inkubationsmedium gespült. Versuchsmedium (bestehend aus dem Inkubationsmedium mit Zusatz von SOD (30 U/ml), Katalase (290 U/ml), Indomethacin (3,5 µg/ml), OxyHb (ca. 8 µM) und Testsubstanz (0,05-250 µM)) wird in die Näpfe gegeben (1 ml/Napf bei 24-Napfplatte). Als Kontrolle wird das konditionierte Versuchsmedium nur mit dem Lösungsmittel der Testsubstanz versetzt. Die mit Versuchsmedium beschickten Platten werden 120 Minuten im Brutschrank bei 37°C, 5 % $CO_2$ und 95 % Luftfeuchtigkeit inkubiert. Nach der 120-minütigen Inkubation wird das Versuchsmedium in Kunststoff-Halbmikroküvetten pipettiert und die Extinktionsdifferenz 576-592 nm am DU 70 gemessen.

Je 2 Näpfe werden mit einer Testsubstanz beschickt (n = 2), auf jeder Platte wird eine Kontrolle unstimuliert, eine Stimulationskontrolle und eine Positiv-Kontrolle (Nitro-L-Arginin, 500 µM vergleichend zu den Testsubstanzen untersucht. Als Maßangabe für die Testsubstanzen wird die Extinktionsdifferenz 576-592 nm prozentual zur Stimulationskontrolle verglichen. Von dem mit Testsubstanz versetzten Versuchsmedium wird zum Zeitpunkt 0 die Extinktionsdifferenz 576-592 nm gemessen sowie der pH-Wert und die Osmolalität bestimmt.

Nach dem Abpipettieren des Versuchsmediums wird die Cytotoxizität der Testsubstanz geprüft (Zellviabilität).

Krankheiten, die durch einen erhöhten NO-Spiegel entstehen und die somit erfindungsgemäß mit den Verbindungen der allgemeinen Formel I behandelt werden können bzw. denen mit diesen vorgebeugt werden kann, sind insbesondere pathologische Blutdruckabfälle, wie sie beim septischen oder hämorrhagischen Schock, bei der Tumor- bzw. Krebstherapie mit Cytokinen oder bei der Leberzirrhose auftreten. Des weiteren entzündliche Erkrankungen, wie rheumatoide Arthritis und insbesondere Colitis ulcerosa, sowie Insulin-abhängiger Diabetes mellitus und Transplantat-Abstoßungsreaktionen.

Aber auch die folgenden Erkrankungen stehen im Zusammenhang mit einer gesteigerten Produktion von Stickstoffmonoxid und können erfindungsgemäß behandelt bzw. vorgebeugt werden. Im Bereich Herz-Kreislauf sind dies Arteriosklerose, postischämische Gewebeschäden und Infarktschäden, Reperfusionsschäden, Myokarditis auf der Grundlage einer Coxsackie-Virus-Infektion und Kardiomyopathie; im Bereich Nervensystem/Zentrales Nervensystem Neuritiden unterschiedlicher Ätiogenese (Neuritisformen), Enzephalomyelitiden, virale neurodegenerative Erkrankungen, Morbus Alzheimer, Hyperalgesie, Epilepsie, Migräne sowie Opioid-Entzugssymptome; im Bereich Niere akutes Nierenversagen sowie Nephritiden unterschiedlicher Ätiogenese, speziell Glomerulonephritis.

Außerdem sind auch Behandlungen im Bereich des Magens und des Uterus/der Plazenta sowie eine Beeinflussung der Motilität der Spermien Anwendungsgebiete für die Verbindungen der allgemeinen Formel I.

Die Verbindungen der allgemeinen Formel I und ihre pharmakologisch annehmbaren Salze können als Hilfsstoffe in biochemischen und pharmakologischen Untersuchungen in der Forschung und in Diagnoseverfahren eingesetzt werden, und sie können am Tier, bevorzugt am Säugetier, und insbesondere am Menschen als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der allgemeinen Formel I oder eines Salzes davon, neben üblichen pharmazeutisch einwandfreien Träger- und Zusatzstoffen, enthalten.

Die Heilmittel können oral, z.B. in Form von Pillen, Tabletten, Lacktabletten, Dragees, Hart- und Weichgelatinekapseln, Lösungen, Sirupen, Emulsionen oder Suspensionen oder Aerosolmischungen verabreicht werden. Die Verabreichung kann aber auch rektal, z.B. in Form von Suppositorien, oder parenteral, z.B. in Form von Injektionslösungen oder Infusionslösungen, oder perkutan, z.B. in Form von Salben oder Tinkturen, erfolgen.

Die pharmazeutischen Präparate können neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmarks- oder Aromatisierungs-Mittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der allgemeinen Formel I oder ihrer pharmakologisch annehmbaren Salze und noch andere therapeutisch wirksame Stoffe enthalten.

Derartige andere therapeutisch wirksame Substanzen sind beispielsweise: β-Rezeptorenblocker, wie z.B. Propranolol, Pindolol, Metoprolol; Vasodilatatoren, wie z.B. Carbocromen; Beruhigungsmittel, wie z.B. Barbitursäurederivate, 1,4-Benzodiazepine und Meprobamat; Diuretica, wie z.B. Chlorothiazid; das Herz tonisierende Mittel, wie z.B. Digitalispräparate; blutdrucksenkende Mittel, wie z.B. Hydralazin, Dihydralazin, Ramipril, Prazosin, Clonidin, Rauwolfia-Alkaloide; Mittel, die den Fettsäurespiegel im Blut senken, wie z.B. Bezafibrat, Fenofibrat; Mittel für die Thromboseprophylaxe, wie z.B. Phenprocoumon; entzündungshemmende Substanzen, wie etwa Corticosteroide, Salicylate oder Propionsäurederivate, wie beispielsweise Ibuprofen; Antibiotika, wie z.B. Penicilline oder Cephalosporine; NO-Donoren, wie z.B. organische Nitrate oder Sydnonimine oder Furoxane.

Die Dosierung kann innerhalb weiter Grenzen variieren und ist in jedem einzelnen Fall den individuellen Gegebenheiten anzupassen. Im allgemeinen ist bei oraler Verabreichung pro menschlichem Individuum eine Tagesdosis von etwa 0,5 bis 100 mg, vorzugsweise 1 bis 20 mg, angemessen. Auch bei anderen Applikationsformen liegt die Tagesdosis, in ähnlichen Mengenbereichen, d.h. im allgemeinen ebenfalls bei 0,5 bis 100 mg/Mensch. Die Tagesdosis kann in mehrere, z.B. 2 bis 4, Teilverabreichungen aufgeteilt werden.

Zur Herstellung der pharmazeutischen Präparate können pharmazeutisch inerte anorganische oder organische Trägerstoffe verwendet werden. Für die Herstellung von Pillen, Tabletten, Dragees und Hartgelatinekapseln kann man z.B. Lactose, Maisstärke oder Derivate davon, Talk, Stearinsäure oder deren Salze etc. verwenden. Trägerstoffe für Weichgelatinekapseln und Suppositorien sind z.B. Fette, Wachse, halbfeste und flüssige Polyole, natürliche oder gehärtete Öle etc. Als Trägerstoffe für die Herstellung von Lösungen und Sirupen eignen sich z.B. Wasser, Saccharose, Invertzucker, Glukose, Polyole etc. Als Trägerstoffe für die Herstellung von Injektionslösungen eignen sich z.B. Wasser, Alkohole, Glyzerin, Polyole oder pflanzliche Öle.

Beispiel 1

a) Die Synthese von 2-Amino-5,6-dihydro-4H-1,3-thiazin-Hydrobromid erfolgte entsprechend Schöberl et al., Liebigs Ann. Chem. 614 (1958), 83, ausgehend von γ-Brompropylamin-Hydrobromid über die Zwischenstufe des γ-Rhodanpropylamins. Die physikalischen Daten entsprachen den dortigen Angaben. 1H-NMR (DMSO, 360 MHz) : 2,02 ppm (quintett, 2H, $H^5$); 3,20 ppm (t, 2H, $H^6$); 3,40 ppm (t, 2H, $H^4$); 8,73 ppm (s, 2H, $NH_2$); 9,70 ppm (s, 1H, NH).

b) Die pharmakologische Wirksamkeit wurde wie oben beschrieben durch NO-Messung mittels Oxyhämoglobin auf Makrophagen bestimmt. Der $IC_{50}$-Wert betrug $1{,}3 \cdot 10^{-6}$ M.

Vergleichsbeispiel

a) Zum Vergleich wurde 2-Amino-4,5-dihydro-1,3-thiazol (Firma Aldrich) in Essigsäureethylester durch Zugabe von etherischer Salzsäure als Hydrochlorid gefällt. Die physikalischen Daten der Substanz stimmten mit den Literaturdaten überein. 1H-NMR (DMSO, 360 MHz) : 3,52 (t, 2H, $H^5$); 3,86 (t, 2H, $H^4$); 9,80 (s, 3H, NH).

b) Die pharmakologische Wirksamkeit wurde gemäß Beispiel 1b) bestimmt. Der $IC_{50}$-Wert betrug $6{,}3 \cdot 10^{-5}$ M.

Beispiel 2

2-Amino-6-phenyl-5,6-dihydro-4H-1,3-thiazin-Hydrochlorid

Zu einer Lösung von 7 g (46 mmol) 3-Amino-1-phenyl-1-propanol (S. L. Meisel et al., J. Am. Chem. Soc. 78 (1956), 4782) in 15 ml Ethanol wird eine Lösung von 5,3 g (46 mmol) tert.-Butylisothiocyanat in 15 ml Ethanol getropft. Das Gemisch wird 30 min unter Rückfluß erhitzt und nach Abkühlen am Rotationsverdampfer eingeengt. Der Rückstand wird in 25 ml konz. Salzsäure aufgenommen und 30 min unter Rückfluß erhitzt. Anschließend wird mit Wasser verdünnt und der ausgefallene Niederschlag abgesaugt. Die Mutterlauge wird mit Natronlauge auf pH 10 gestellt und mit Essigsäureethylester extrahiert. Die vereinigten organischen Phasen werden getrocknet und mit etherischer Salzsäure versetzt. Der ausfallende Niederschlag wird abgesaugt. Die vereinigten Niederschläge werden aus Isopropanol/Hexan umkristallisiert. Ausbeute: 3 g weiße Kristalle vom Schmp. 205-206°C (H. W. Schubert und O. Behner, Arch. Pharm. 301 (1968), 750: Schmp. 202-204°C).

Beispiel 3

2-Amino-4,4-dimethyl-5,6-dihydro-1,3-thiazin-Hydrochlorid

a) Die Substanz ist analog Beispiel 2 erhältlich. Weiße Kristalle vom Schmp. 153°C.

b) Die pharmakologische Wirkung wurde in dem oben beschriebenen Aktivitätsassy ermittelt, bei dem das von der NO-Synthase als Coprodukt gebildete Citrullin bestimmt wird. Es wurden folgende Hemmungen der induzierbaren NO-Synthase beobachtet:

| | | | |
|---|---|---|---|
| Konzentration: | 10 µM | Restaktivität: | 12 % |
| | 1 µM | | 15 % |

Die nachfolgenden Beispiele betreffen pharmazeutische Zubereitungsformen.

**Beispiel A**

Gelatineweichkapseln, enthaltend 100 mg Wirkstoff pro Kapsel:

| | **pro Kapsel** |
|---|---|
| Wirkstoff | 100 mg |
| aus Kokosfett fraktioniertes Triclycerid-Gemisch | 400 mg |
| Kapselinhalt | 500 mg |

**Beispiel B**

Injektionslösung, enthaltend 2,0 mg Wirkstoff pro ml:

| | **pro ml** |
|---|---|
| Wirkstoff | 2,0 mg |
| Polyethylenglycol 400 | 5,0 mg |
| Natriumchlorid | 2,7 mg |
| Wasser zu Injektionszwecken | ad 1 ml |

**Beispiel C**

Emulsion, enthaltend 60 mg Wirkstoff pro 5 ml:

| | pro 100 ml Emulsion |
|---|---|
| Wirkstoff | 1,2 g |
| Neutralöl | q.s. |
| Natriumcarboxymethylcellulose | 0,6 g |
| Polyoxyethylen-stearat | q.s. |
| Glycerin rein | 0,2 bis 2,0 g |
| Geschmacksstoff | q.s. |
| Wasser (entsalzt oder destilliert) | ad 100 ml |

**Beispiel D**

Rektale Arzneiform, enthaltend 40 mg Wirkstoff pro Suppositorium:

| | pro Suppositorium |
|---|---|
| Wirkstoff | 40 mg |
| Suppositoriengrundmasse | ad 2 g |

**Beispiel E**

Tabletten, enthaltend 40 mg Wirkstoff pro Tablette:

| | pro Tablette |
|---|---|
| Wirkstoff | 40 mg |
| Lactose | 600 mg |
| Maisstärke | 300 mg |
| lösliche Stärke | 20 mg |
| Magnesiumstearat | 40 mg |
| | 1000 mg |

**Beispiel F**

Dragees, enthaltend 50 mg Wirkstoff pro Dragee:

| | pro Dragee |
|---|---|
| Wirkstoff | 50 mg |
| Maisstärke | 100 mg |
| Lactose | 60 mg |
| sec. Calciumphosphat | 30 mg |
| lösliche Stärke | 5 mg |
| Magnesiumstearat | 10 mg |
| kolloidale Kieselsäure | 5 mg |
| | 260 mg |

**Beispiel G**

Für die Herstellung des Inhalts von Hartgelatinekapseln eignen sich die folgenden Rezepturen:

| | | |
|---|---|---|
| a) | Wirkstoff | 100 mg |
| | Maisstärke | 300 mg |
| | | 400 mg |
| b) | Wirkstoff | 140 mg |
| | Milchzucker | 180 mg |
| | Maisstärke | 180 mg |
| | | 500 mg |

**Beispiel H**

Tropfen können nach folgender Rezeptur hergestellt werden (100 mg Wirkstoff in 1 ml = 20 Tropfen):

| | |
|---|---|
| Wirkstoff | 10 g |
| Benzoesäuremethylester | 0,07 g |
| Benzoesäureethylester | 0,03 g |
| Ethanol 96 %ig | 5 ml |
| entmineralisiertes Wasser | ad 100 ml |

## Patentansprüche

1. Verwendung von 2-Amino-1,3-thiazinen der allgemeinen Formel I,

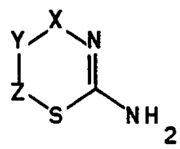

(I)

in der X für $CR^1R^2$ steht und
$R^1$ und $R^2$ unabhängig voneinander für Wasserstoff, Halogen, $R^3$, $R^4$, $OR^5$, $SR^{5'}$, $S(O)_p$-$R^6$, wobei p für 1 oder 2 steht, $NR^7R^8$, Cyan, Nitro, $COOR^9$, $CONR^{7'}R^{8'}$ oder $P(O)(OH)_2$ stehen; oder $R^1$ und $R^2$ zusammen für ($C_1$-$C_6$)-Alkyliden oder ($C_3$-$C_7$)-Cycloalkyliden stehen, die auch jeweils einen oder mehrere Reste $R^{10}$ tragen können, oder $R^1$ und $R^2$ zusammen mit dem Kohlenstoffatom, an das sie gebunden sind, einen Rest der allgemeinen Formel II

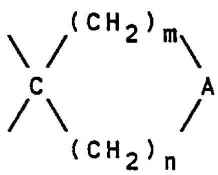

(II)

bilden, der vollständig oder teilweise dehydriert sein kann, der auch benzanelliert sein kann, der durch einen oder mehrere gleiche oder verschiedene Reste $R^{10}$ substituiert sein kann und worin m und n unabhängig voneinander für die Zahlen 0, 1, 2, 3, 4 oder 5 stehen, aber die Summe m + n einen der Werte 1, 2, 3, 4 oder 5 hat;
$R^3$ für ($C_1$-$C_{10}$)-Alkyl, ($C_2$-$C_{10}$)-Alkenyl, ($C_2$-$C_{10}$)-Alkinyl; ($C_3$-$C_7$)-Cycloalkyl, ($C_9$-$C_{11}$)-Benzocycloalkyl oder den Rest eines 4- bis 10-gliedrigen Heterocyclus mit einem bis vier gleichen oder verschiedenen Heteroatomen aus der Reihe Stickstoff, Sauerstoff und Schwefel steht, der aromatisch, teilweise gesättigt oder vollständig gesättigt sein kann, wobei der Alkyl-, Alkenyl-, Alkinyl-, Cycloalkyl- und der heterocyclische Rest durch einen oder mehrere gleiche oder verschiedene Reste $R^{10}$ substituiert sein können;
$R^4$ für Phenyl oder Naphthyl steht, die durch ein bis drei gleiche oder verschiedene Reste aus der Reihe ($C_1$-$C_4$)-Alkyl, ($C_1$-$C_4$)-Alkoxy, Halogen, Nitro, Trifluormethyl, Cyan, Hydroxy, ($C_1$-$C_4$)-Alkoxycarbonyl, Carboxyl, Carbamoyl, ($C_1$-$C_4$)-Alkyl-$S(O)_q$, wobei q für 0, 1 oder 2 steht, Amino, ($C_1$-$C_4$)-Alkylcarbonylamino, ($C_1$-$C_4$)-Alkylamino und Di(($C_1$-$C_4$)-Alkyl)amino substituiert sein können;
$R^5$ und $R^{5'}$ unabhängig voneinander für Wasserstoff, ($C_1$-$C_{10}$)-Alkyl, in dem die Alkylkette auch durch ein bis drei Sauerstoffatome unterbrochen sein kann, ($C_2$-$C_{10}$)-Alkenyl, ($C_3$-$C_7$)-Cycloalkyl, $R^4$, $R^4$-($C_1$-$C_6$)-Alkyl, den Rest eines 5- bis 7-gliedrigen Heterocyclus mit einem bis vier gleichen oder verschiedenen Heteroatomen aus der Reihe Stickstoff, Sauerstoff und Schwefel steht, der aromatisch, teilweise gesättigt oder vollständig gesättigt sein kann, ($C_1$-$C_{10}$)-Alkanoyl, $R^4$-CO, $R^4$-($C_1$-$C_6$)-Alkyl-CO, ($C_1$-$C_{10}$)-Alkyloxycarbonyl, $R^4$O-CO, $R^4$-($C_1$-$C_6$)-Alkoxy-CO oder einen Rest der allgemeinen Formel III

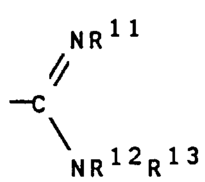

(III)

stehen;
$R^6$ für ($C_1$-$C_{10}$)-Alkyl, ($C_2$-$C_{10}$)-Alkenyl, $R^4$ oder $R^4$-($C_1$-$C_6$)-Alkyl steht;
$R^7$ und $R^8$ unabhängig voneinander die für $R^5$ angegebenen Bedeutungen haben oder für ($C_1$-$C_6$)-Alkylsulfonyl

oder $R^4$-$S(O)_2$ stehen oder $R^7$ und $R^8$ zusammen mit dem Stickstoffatom, an das sie gebunden sind, einen Rest der allgemeinen Formel IV

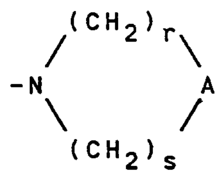

(IV)

bilden, der an den $CH_2$-Gruppen durch einen oder mehrere gleiche oder verschiedene Reste $R^{10}$ substituiert sein kann und worin r und s unabhängig voneinander für die Zahlen 1, 2, 3 oder 4 stehen, aber die Summe r + s kleiner als 6 ist;
$R^{7'}$ und $R^{8'}$ unabhängig von $R^7$ und $R^8$ die für $R^7$ und $R^8$ angegebenen Bedeutungen haben;
$R^9$ eine der Bedeutungen von $R^5$ hat, aber nicht für den Rest der allgemeinen Formel III steht;
$R^{10}$ für ($C_1$-$C_6$)-Alkyl, ($C_3$-$C_7$)-Cycloalkyl, den Rest eines 5- bis 7-gliedrigen Heterocyclus mit einem bis drei gleichen oder verschiedenen Heteroatomen aus der Reihe Stickstoff, Sauerstoff und Schwefel, der aromatisch, teilweise gesättigt oder vollständig gesättigt sein kann, $R^4$, Halogen, $OR^5$, $SR^{5'}$, $S(O)_p$-$R^6$, wobei p für 1 oder 2 steht, $NR^7R^8$, die Hydroximinogruppe, die Oxogruppe, Cyan, Nitro, $COOR^9$, $CONR^{7'}R^{8'}$ oder $P(O)(OH)_2$ steht;
die Reste $R^{11}$, $R^{12}$ und $R^{13}$ unabhängig voneinander für Wasserstoff, ($C_1$-$C_6$)-Alkyl, ($C_3$-$C_7$)-Cycloalkyl, $R^4$, ($C_1$-$C_6$)-Alkanoyl, ($C_1$-$C_6$)-Alkyl-$S(O)_2$ oder $R^4$-$S(O)_2$ stehen;
$R^{14}$ für ($C_1$-$C_4$)-Alkyl, Benzyl, Phenyl, ($C_1$-$C_6$)-Alkanoyl, ($C_1$-$C_6$)-Alkyl-$S(O)_2$ oder $R^4$-$S(O)_2$ steht;
A für eine Methylengruppe, die durch einen oder zwei gleiche oder verschiedene Reste $R^{10}$ substituiert sein kann, für Sauerstoff, Schwefel oder $NR^{14}$ steht;
Y für $CR^{20}R^{21}$ steht und $R^{20}$ und $R^{21}$ unabhängig von den Bedeutungen der Reste $R^1$ und $R^2$ die für $R^1$ und $R^2$ angegebenen Bedeutungen haben oder zusammen für =O, =N-OH oder =S stehen;
Z für $CR^{22}R^{23}$ steht und $R^{22}$ und $R^{23}$ unabhängig von den Bedeutungen der Reste $R^1$ und $R^2$ die für $R^1$ und $R^2$ angegebenen Bedeutungen haben;
wobei auch Reste der Gruppen X, Y und Z über eine Bindung verknüpft sein können, so daß ein Bicyclus oder Tricyclus gebildet wird;
und ihrer tautomeren Formen sowie ihrer pharmakologisch verträglichen Salze zur Vorbeugung und Behandlung von Krankheiten, die durch einen erhöhten Stickstoffmonoxid-Spiegel bedingt sind.

2. Verwendung gemäß Anspruch 1, dadurch gekennzeichnet, daß X, Y und Z in der allgemeinen Formel I für die $CH_2$-Gruppe stehen.

3. Verwendung gemaß Anspruch 1 und/oder 2 zur Behandlung und Vorbeugung pathologischer Blutdruckabfälle, insbesondere beim septischen Schock und der Krebstherapie mit Cytokinen.

4. Verwendung gemäß Anspruch 1 und/oder 2 zur Behandlung und Vorbeugung von entzündlichen Erkrankungen, insbesondere Colitis ulcerosa.

5. Verwendung gemäß Anspruch 1 und/oder 2 zur Behandlung und Vorbeugung von Infarktschäden und/oder Reperfusionsschäden.

6. Verwendung gemäß Anspruch 1 und/oder 2 zur Behandlung und Vorbeugung von Transplantat-Abstoßungsreaktionen.

7. Verwendung gemäß Anspruch 1 und/oder 2 zur Behandlung und Vorbeugung von Erkrankungen des Nervensystems aus der Reihe Morbus Alzheimer, Epilepsie und Migräne.

**8.** 2-Amino-1,3-thiazine der allgemeinen Formel I

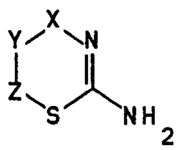

(I)

in der X für $CR^1R^2$ steht und

$R^1$ und $R^2$ unabhängig voneinander für Wasserstoff, Halogen, $R^3$, $R^4$, $OR^5$, $SR^{5'}$, $S(O)_p$-$R^6$, wobei p für 1 oder 2 steht, $NR^7R^8$, Cyan, Nitro, $COOR^9$, $CONR^{7'}R^{8'}$ oder $P(O)(OH)_2$ stehen; oder $R^1$ und $R^2$ zusammen für ($C_1$-$C_6$)-Alkyliden oder ($C_3$-$C_7$)-Cycloalkyliden stehen, die auch jeweils einen oder mehrere Reste $R^{10}$ tragen können, oder $R^1$ und $R^2$ zusammen mit dem Kohlenstoffatom, an das sie gebunden sind, einen Rest der allgemeinen Formel II

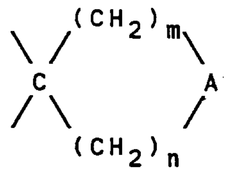

(II)

bilden, der vollständig oder teilweise dehydriert sein kann, der auch benzanelliert sein kann, der durch einen oder mehrere gleiche oder verschiedene Reste $R^{10}$ substituiert sein kann und worin m und n unabhängig voneinander für die Zahlen 0, 1, 2, 3, 4 oder 5 stehen, aber die Summe m + n einen der Werte 1, 2, 3, 4 oder 5 hat;

$R^3$ für ($C_1$-$C_{10}$)-Alkyl, ($C_2$-$C_{10}$)-Alkenyl, ($C_2$-$C_{10}$)-Alkinyl; ($C_3$-$C_7$)-Cycloalkyl, ($C_9$-$C_{11}$)-Benzocycloalkyl oder den Rest eines 4- bis 10-gliedrigen Heterocyclus mit einem bis vier gleichen oder verschiedenen Heteroatomen aus der Reihe Stickstoff, Sauerstoff und Schwefel steht, der aromatisch, teilweise gesättigt oder vollständig gesättigt sein kann, wobei der Alkyl-, Alkenyl-, Alkinyl-, Cycloalkyl- und der heterocyclische Rest durch einen oder mehrere gleiche oder verschiedene Reste $R^{10}$ substituiert sein können;

$R^4$ für Phenyl oder Naphthyl steht, die durch ein bis drei gleiche oder verschiedene Reste aus der Reihe ($C_1$-$C_4$)-Alkyl, ($C_1$-$C_4$)-Alkoxy, Halogen, Nitro, Trifluormethyl, Cyan, Hydroxy, ($C_1$-$C_4$)-Alkoxycarbonyl, Carboxyl, Carbamoyl, ($C_1$-$C_4$)-Alkyl-$S(O)_q$, wobei q für 0, 1 oder 2 steht, Amino, ($C_1$-$C_4$)-Alkylcarbonylamino, ($C_1$-$C_4$)-Alkylamino und Di(($C_1$-$C_4$)-Alkyl)amino substituiert sein können;

$R^5$ und $R^{5'}$ unabhängig voneinander für Wasserstoff, ($C_1$-$C_{10}$)-Alkyl, in dem die Alkylkette auch durch ein bis drei Sauerstoffatome unterbrochen sein kann, ($C_2$-$C_{10}$)-Alkenyl, ($C_3$-$C_7$)-Cycloalkyl, $R^4$, $R^4$-($C_1$-$C_6$)-Alkyl, den Rest eines 5- bis 7-gliedrigen Heterocyclus mit einem bis vier gleichen oder verschiedenen Heteroatomen aus der Reihe Stickstoff, Sauerstoff und Schwefel steht, der aromatisch, teilweise gesättigt oder vollständig gesättigt sein kann, ($C_1$-$C_{10}$)-Alkanoyl, $R^4$-CO, $R^4$-($C_1$-$C_6$)-Alkyl-CO, ($C_1$-$C_{10}$)-Alkyloxycarbonyl, $R^4$O-CO, $R^4$-($C_1$-$C_6$)-Alkoxy-CO oder einen Rest der allgemeinen Formel III

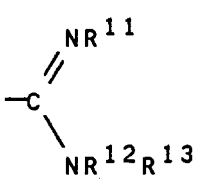

(III)

stehen;

$R^6$ für ($C_1$-$C_{10}$)-Alkyl, ($C_2$-$C_{10}$)-Alkenyl, $R^4$ oder $R^4$-($C_1$-$C_6$)-Alkyl steht;

$R^7$ und $R^8$ unabhängig voneinander die für $R^5$ angegebenen Bedeutungen haben oder für ($C_1$-$C_6$)-Alkylsulfonyl oder $R^4$-$S(O)_2$ stehen oder $R^7$ und $R^8$ zusammen mit dem Stickstoffatom, an das sie gebunden sind, einen Rest der allgemeinen Formel IV

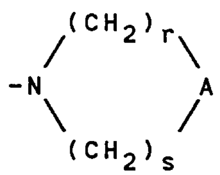

(IV)

bilden, der an den $CH_2$-Gruppen durch einen oder mehrere gleiche oder verschiedene Reste $R^{10}$ substituiert sein kann und worin r und s unabhängig voneinander für die Zahlen 1, 2, 3 oder 4 stehen, aber die Summe r + s kleiner als 6 ist;

$R^{7'}$ und $R^{8'}$ unabhängig von $R^7$ und $R^8$ die für $R^7$ und $R^8$ angegebenen Bedeutungen haben;

$R^9$ eine der Bedeutungen von $R^5$ hat, aber nicht für den Rest der allgemeinen Formel III steht;

$R^{10}$ für ($C_1$-$C_6$)-Alkyl, ($C_3$-$C_7$)-Cycloalkyl, den Rest eines 5- bis 7-gliedrigen Heterocyclus mit einem bis drei gleichen oder verschiedenen Heteroatomen aus der Reihe Stickstoff, Sauerstoff und Schwefel, der aromatisch, teilweise gesättigt oder vollständig gesättigt sein kann, $R^4$, Halogen, $OR^5$, $SR^{5'}$, $S(O)_p$-$R^6$, wobei p für 1 oder 2 steht, $NR^7R^8$, die Hydroximinogruppe, die Oxogruppe, Cyan, Nitro, $COOR^9$, $CONR^{7'}R^{8'}$ oder $P(O)(OH)_2$ steht;

die Reste $R^{11}$, $R^{12}$ und $R^{13}$ unabhängig voneinander für Wasserstoff, ($C_1$-$C_6$)-Alkyl, ($C_3$-$C_7$)-Cycloalkyl, $R^4$, ($C_1$-$C_6$)-Alkanoyl, ($C_1$-$C_6$)-Alkyl-$S(O)_2$ oder $R^4$-$S(O)_2$ stehen;

$R^{14}$ für ($C_1$-$C_4$)-Alkyl, Benzyl, Phenyl, ($C_1$-$C_6$)-Alkanoyl, ($C_1$-$C_6$)-Alkyl-$S(O)_2$ oder $R^4$-$S(O)_2$ steht;

A für eine Methylengruppe, die durch einen oder zwei gleiche oder verschiedene Reste $R^{10}$ substituiert sein kann, für Sauerstoff, Schwefel oder $NR^{14}$ steht;

Y für $CR^{20}R^{21}$ steht und $R^{20}$ und $R^{21}$ unabhängig von den Bedeutungen der Reste $R^1$ und $R^2$ die für $R^1$ und $R^2$ angegebenen Bedeutungen haben oder zusammen für =O, =N-OH oder =S stehen;

Z für $CR^{22}R^{23}$ steht und $R^{22}$ und $R^{23}$ unabhängig von den Bedeutungen der Reste $R^1$ und $R^2$ die für $R^1$ und $R^2$ angegebenen Bedeutungen haben;

wobei auch Reste der Gruppen X, Y und Z über eine Bindung verknüpft sein können, so daß ein Bicyclus oder Tricyclus gebildet wird;

und ihrer tautomeren Formen sowie ihrer pharmakologisch verträglichen Salze als pharmakologische Wirkstoffe, wobei aber $R^1$, $R^2$, $R^{20}$, $R^{21}$, $R^{22}$ und $R^{23}$ nicht gleichzeitig für Wasserstoff stehen können und, wenn $R^1$, $R^2$, $R^{20}$, $R^{21}$ und $R^{23}$ für Wasserstoff stehen, $R^{22}$ nicht für Phenyl stehen kann, und für den Fall, daß $R^{20}$, $R^{21}$, $R^{22}$ und $R^{23}$ für Wasserstoff stehen und $R^1$ und $R^2$ zusammen mit dem Kohlenstoffatom, an das sie gebunden sind, einen Rest der allgemeinen Formel II bilden, A nicht für Sauerstoff stehen kann, wenn m oder n 0 bedeutet und die Summe m + n 3 oder 4 ist.

9. 2-Amino-1,3-thiazine der allgemeinen Formel I,

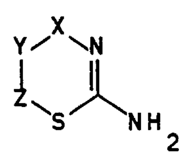

(I)

in der X für $CR^1R^2$ steht und

$R^1$ und $R^2$ unabhängig voneinander für Wasserstoff, Halogen, $R^3$, $R^4$, $OR^5$, $SR^{5'}$, $S(O)_p$-$R^6$, wobei p für 1 oder 2 steht, $NR^7R^8$, Cyan, Nitro, $COOR^9$, $CONR^{7'}R^{8'}$ oder $P(O)(OH)_2$ stehen; oder $R^1$ und $R^2$ zusammen für ($C_1$-$C_6$)-Alkyliden oder ($C_3$-$C_7$)-Cycloalkyliden stehen, die auch jeweils einen oder mehrere Reste $R^{10}$ tragen können, oder $R^1$ und $R^2$ zusammen mit dem Rohlenstoffatom, an das sie gebunden sind, einen Rest der allgemeinen Formel II

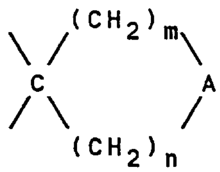 (II)

bilden, der vollständig oder teilweise dehydriert sein kann, der auch benzanelliert sein kann, der an den $CH_2$-Gruppen durch einen oder mehrere gleiche oder verschiedene Reste $R^{10}$ substituiert sein kann und worin m und n unabhängig voneinander für die Zahlen 0, 1, 2, 3, 4 oder 5 stehen, aber die Summe m + n einen der Werte 1, 2, 3, 4 oder 5 hat;

$R^3$ für $(C_1$-$C_{10})$-Alkyl, $(C_2$-$C_{10})$-Alkenyl, $(C_2$-$C_{10})$-Alkinyl; $(C_3$-$C_7)$-Cycloalkyl, $(C_9$-$C_{11})$-Benzocycloalkyl oder den Rest eines 4-bis 10-gliedrigen Heterocyclus mit einem bis vier gleichen oder verschiedenen Heteroatomen aus der Reihe Stickstoff, Sauerstoff und Schwefel steht, der aromatisch, teilweise gesättigt oder vollständig gesättigt sein kann, wobei der Alkyl-, Alkenyl-, Alkinyl-, Cycloalkyl- und der heterocyclische Rest durch einen oder mehrere gleiche oder verschiedene Reste $R^{10}$ substituiert sein können;

$R^4$ für Phenyl oder Naphthyl steht, die durch ein bis drei gleiche oder verschiedene Reste aus der Reihe $(C_1$-$C_4)$-Alkyl, $(C_1$-$C_4)$-Alkoxy, Halogen, Nitro, Trifluormethyl, Cyan, Hydroxy, $(C_1$-$C_4)$-Alkoxycarbonyl, Carboxyl, Carbamoyl, $(C_1$-$C_4)$-Alkyl-$S(O)_q$, wobei q für 0, 1 oder 2 steht, Amino, $(C_1$-$C_4)$-Alkylcarbonylamino, $(C_1$-$C_4)$-Alkylamino und Di($(C_1$-$C_4)$-Alkyl)amino substituiert sein können;

$R^5$ und $R^{5'}$ unabhängig voneinander für Wasserstoff, $(C_1$-$C_{10})$-Alkyl, in dem die Alkylkette auch durch ein bis drei Sauerstoffatome unterbrochen sein kann, $(C_2$-$C_{10})$-Alkenyl, $(C_3$-$C_7)$-Cycloalkyl, $R^4$, $R^4$-$(C_1$-$C_6)$-Alkyl, den Rest eines 5- bis 7-gliedrigen Heterocyclus mit einem bis vier gleichen oder verschiedenen Heteroatomen aus der Reihe Stickstoff, Sauerstoff und Schwefel steht, der aromatisch, teilweise gesättigt oder vollständig gesättigt sein kann, $(C_1$-$C_{10})$-Alkanoyl, $R^4$-CO, $R^4$-$(C_1$-$C_6)$-Alkyl-CO, $(C_1$-$C_{10})$-Alkyloxycarbonyl, $R^4$O-CO, $R^4$-$(C_1$-$C_6)$-Alkoxy-CO oder einen Rest der allgemeinen Formel III

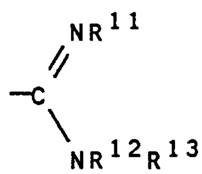 (III)

stehen;

$R^6$ für $(C_1$-$C_{10})$-Alkyl, $(C_2$-$C_{10})$-Alkenyl, $R^4$ oder $R^4$-$(C_1$-$C_6)$-Alkyl steht;

$R^7$ und $R^8$ unabhängig voneinander die für $R^5$ angegebenen Bedeutungen haben oder für $(C_1$-$C_6)$-Alkylsulfonyl oder $R^4$-$S(O)_2$ stehen oder $R^7$ und $R^8$ zusammen mit dem Stickstoffatom, an das sie gebunden sind, einen Rest der allgemeinen Formel IV

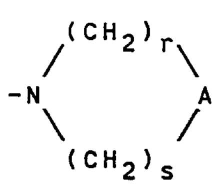 (IV)

bilden, der an den $CH_2$-Gruppen durch einen oder mehrere gleiche oder verschiedene Reste $R^{10}$ substituiert sein kann und worin r und s unabhängig voneinander für die Zahlen 1, 2, 3 oder 4 stehen, aber die Summe r + s kleiner als 6 ist;

$R^{7'}$ und $R^{8'}$ unabhängig von $R^7$ und $R^8$ die für $R^7$ und $R^8$ angegebenen Bedeutungen haben;

$R^9$ eine der Bedeutungen von $R^5$ hat, aber nicht für den Rest der allgemeinen Formel III steht;

$R^{10}$ für ($C_1$-$C_6$)-Alkyl, ($C_3$-$C_7$)-Cycloalkyl, den Rest eines 5- bis 7-gliedrigen Heterocyclus mit einem bis drei gleichen oder verschiedenen Heteroatomen aus der Reihe Stickstoff, Sauerstoff und Schwefel, der aromatisch, teilweise gesättigt oder vollständig gesättigt sein kann, $R^4$, Halogen, $OR^5$, $SR^{5'}$, $S(O)_p$-$R^6$, wobei p für 1 oder 2 steht, $NR^7R^8$, die Hydroximinogruppe, die Oxogruppe, Cyan, Nitro, $COOR^9$, $CONR^{7'}R^{8'}$ oder $P(O)(OH)_2$ steht;
die Reste $R^{11}$, $R^{12}$ und $R^{13}$ unabhängig voneinander für Wasserstoff, ($C_1$-$C_6$)-Alkyl, ($C_3$-$C_7$)-Cycloalkyl, $R^4$, ($C_1$-$C_6$)-Alkanoyl, ($C_1$-$C_6$)-Alkyl-$S(O)_2$ oder $R^4$-$S(O)_2$ stehen;
$R^{14}$ für ($C_1$-$C_4$)-Alkyl, Benzyl, Phenyl, ($C_1$-$C_6$)-Alkanoyl, ($C_1$-$C_6$)-Alkyl-$S(O)_2$ oder $R^4$-$S(O)_2$ steht;
A für eine Methylengruppe, die durch einen oder zwei gleiche oder verschiedene Reste $R^{10}$ substituiert sein kann, für Sauerstoff, Schwefel oder $NR^{14}$ steht;
Y für $CR^{20}R^{21}$ steht und $R^{20}$ und $R^{21}$ unabhängig von den Bedeutungen der Reste $R^1$ und $R^2$ die für $R^1$ und $R^2$ angegebenen Bedeutungen haben oder zusammen für =O, =N-OH oder =S stehen;
Z für $CR^{22}R^{23}$ steht und $R^{22}$ und $R^{23}$ unabhängig von den Bedeutungen der Reste $R^1$ und $R^2$ die für $R^1$ und $R^2$ angegebenen Bedeutungen haben;
wobei auch Reste der Gruppen X, Y und Z über eine Bindung verknüpft sein können, so daß ein Bicyclus oder Tricyclus gebildet wird;
und ihrer tautomeren Formen sowie ihrer pharmakologisch verträglichen Salze,
wobei aber,

a) wenn $R^{20}$, $R^{21}$, $R^{22}$ und $R^{23}$ für Wasserstoff stehen, nicht

a1) gleichzeitig $R^1$ für Wasserstoff und $R^2$ für Wasserstoff, Methyl, Ethoxy, Carboxy, Phenyl, Chlorphenyl, Dichlorphenyl, Methylphenyl, Dimethylphenyl, Nitrophenyl, Methoxyphenyl oder Acetylaminophenyl stehen kann;
a2) gleichzeitig $R^1$ für Methyl und $R^2$ für Phenyl oder 4-Methoxyphenyl oder gleichzeitig $R^1$ für Ethyl und $R^2$ für 3,4-Dimethoxyphenyl stehen kann;
a3) gleichzeitig $R^1$ und $R^2$ zusammen mit dem Kohlenstoffatom, an das sie gebunden sind, einen unsubstituierten Rest der allgemeinen Formel II bilden können, worin A für eine Methylengruppe und m und n beide für 2 stehen, oder einen Rest der allgemeinen Formel II bilden können, worin A für Sauerstoff und n für 0 steht;
a4) gleichzeitig $R^1$ und $R^2$ zusammen mit dem Kohlenstoffatom, an das sie gebunden sind, einen 6-Methoxy-1,2,3,4-tetrahydronaphthalin-1-yliden-Rest bilden können;

b) wenn $R^1$, $R^2$, $R^{20}$ und $R^{21}$ für Wasserstoff stehen, nicht

b1) gleichzeitig $R^{22}$ für Wasserstoff und $R^{23}$ für Methyl, Phenyl, Methoxyphenyl oder Brommethyl stehen kann;
b2) gleichzeitig $R^{22}$ und $R^{23}$ zusammen für $=CH_2$ stehen können;

c) wenn $R^1$, $R^2$, $R^{22}$ und $R^{23}$ für Wasserstoff stehen, nicht

c1) gleichzeitig $R^{20}$ für Wasserstoff und $R^{21}$ für Chlor, Hydroxy, Methylsulfonyl, n-Butylsulfonyl, Benzoyloxy, Phenyl oder Chlorphenyl stehen kann;

c2) gleichzeitig $R^{20}$ und $R^{21}$ zusammen für =O stehen können;

und wobei nicht gleichzeitig $R^1$, $R^{21}$, $R^{22}$ und $R^{23}$ für Wasserstoff stehen und $R^2$ und $R^{20}$ zusammen mit den beiden Kohlenstoffatomen, an die sie gebunden sind, einen 1,2-Cyclohexylen-Rest bilden können.

**10.** Pharmazeutisches Präparat, dadurch gekennzeichnet, daß es ein oder mehrere der 2-Amino-1,3-thiazine der allgemeinen Formel I gemäß Anspruch 1 und/oder 2 und/oder ein oder mehrere pharmakologisch annehmbare Salze davon als Wirkstoff zusammen mit pharmazeutisch annehmbaren Träger- und Zusatzstoffen und gegebenenfalls noch einen oder mehrere andere pharmakologische Wirkstoffe enthält.

Europäisches Patentamt

# EUROPÄISCHER RECHERCHENBERICHT

Nummer der Anmeldung
EP 95 11 7500

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| P,X | BIOCHEM. BIOPHYS. RES. COMMUN., Bd. 206, Nr. 2, 1995 Seiten 511-517, XP 000565662 M. NAKANE ET AL. 'Functional expression of three isoforms of human nitric oxide synthase in baculovirus-infected insect cells.' * das ganze Dokument * --- | 1 | A61K31/54<br>C07D279/04 |
| P,X | MOL. PHARMACOL., Bd. 47, Nr. 4, 1995 Seiten 831-834, XP 000566159 M. NAKANE ET AL. 'Novel potent and selective inhibitors of inducible nitric oxide synthase.' * das ganze Dokument * --- | 1,4 | |
| P,X | CAN. J. PHYSIOL. PHARMACOL., Bd. 73, Nr. 5, 1995 Seiten 665-669, XP 000566119 W.R. TRACEY ET AL. 'In vivo pharmacological evaluation of the two novel type II inducible nitric oxide synthase inhibitors.' * das ganze Dokument * --- | 1,3,4 | RECHERCHIERTE SACHGEBIETE (Int.Cl.6)<br>A61K<br>C07D |
| P,X | WO-A-95 11231 (G.D. SEARLE & CO) 27.April 1995 * Seite 3; Ansprüche; Beispiel 17 * ----- | 1-5 | |

Der vorliegende Recherchenbericht wurde für alle Patentansprüche erstellt

| Recherchenort | Abschlußdatum der Recherche | Prüfer |
|---|---|---|
| DEN HAAG | 22.März 1996 | Klaver, T |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)